# EUROPEAN PATENT SPECIFICATION

(11) **EP 0 965 351 B1**
(45) Date of publication and mention of the grant of the patent: **01.06.2005**
(21) Application number: 99201794.7
(22) Date of filing: 04.06.1999
(51) Int. Cl.: A23L 1/308

(54) **Food composition**
Nahrungsmittelzusammensetzung
Composition alimentaire

(30) Priority: 16.06.1998 EP 98830365
(43) Date of publication of application: 22.12.1999
(60) Divisional application: 02078716.4
(73) Proprietor: Zohoungbogbo, Mathias Christian, 10040 Rivalta Di Torino (IT); Gobbato, Rosa Anna, 10040 Rivalta di Torino (IT)
(72) Inventor: Zohoungbogbo, Mathias Christian, 10040 Rivalta di Torino (Torino) (IT)
(74) Representative: Long, Giorgio

(56) References cited:
- WO-A-97/39355
- GB-A- 1 508 940
- US-A- 5 106 634
- "Guide to Our Products - Singles, Combinations & Chinese " , [Online] XP002135817 Retrieved from the Internet: <URL:www.pagedepot.com/canada/tnt-herbals/ products.htm> [retrieved on 2000-04-14]

## Description

This invention relates to the sector of the food and dietetic industry.

In particular, the invention relates to a substitute food composition for cereal flours in general and for wheat flour in particular, and foods which can be prepared using this composition.

The invention also relates to a method for controlling the body weight of an individual.

It is well known that in the developed nations and even in the developing nations the percentage of individuals having excess weight if not obesity problems is constantly and progressively increasing. This has important consequences both for the health of individuals and for the overall health cost of the various nations, because it has been amply demonstrated that obesity or even being overweight are important associated causes of cardiovascular and metabolic diseases, such as myocardial infarction, stroke, type II diabetes, etc.

The dietetic measures generally proposed by dieticians to combat and/or prevent excess weight mainly consist of low-calorie or low-fat regimes.

Another widely recommended measure for the control of body weight which is even advocated by the mass media (periodicals, television, etc.) is adoption of the famous "Mediterranean diet", based on foods rich in complex carbohydrates such as pasta, rice, bread and the like.

It is, however, a fact that the widespread use of low-calorie diets and the Mediterranean diet has not in fact resulted in any statistically significant change in the percentage of obese or overweight persons; there is instead a progressive increase in this percentage.

A low-calorie diet can in fact have some temporary effect on reducing body weight but this cannot be maintained for a long period, either because it results in general weakening of the body, or because over the long term it is rejected by the individual because of the monotony of the flavours of the food making it up (essentially meat, fish and greens).

The so-called "Mediterranean diet" is in fact only suitable for maintaining the right weight and the right form in individuals who are engaged in vigorous physical activity. Persons who are engaged in essentially sedentary work can on the other hand experience an increase in weight and an accumulation of lipids when they feed themselves on foods based essentially on carbohydrates.

US-A-5 106 634 discloses a food product comprising milled starch-bearing cereal grains which have had 90 to 100% of the starch enzymatically converted, said grains having a protein content on a dry matter basis substantially between 17 and 35% by weight, a total dietary fibre content on a dry matter basis substantially between 30 to 70% by weight and a nitrogen-free extract content on a dry matter basis less than 40%. Further, the grains have a coating of residual sugars thereon from the enzymatic conversion of starch which is 4 to 30% by weight on a dry matter basis.

GB-A-1 508 940 discloses a low-calorie farinaceous composition comprising from 20 to 75% by weight of modified polydextrose, from 2 to 20% by weight of proteinaceous material, from 10 to 40% by weight of cellulose derivative and from 5 to 20% by weight of flour.

The problem underlying this invention is that of providing a diet/foodstuff which allows obese or merely overweight persons to recover their ideal weight by eliminating excess lipids and to maintain such an ideal weight over a long period of time. All this without the persons having to subject themselves to a low-calorie diet and to suffer the deprivation of foodstuffs which it is difficult to do without over long periods, such as pasta, bread and bakery products in general.

Such a problem is solved according to the invention by a food composition in the form of a flour comprising at least 50% of protein, up to 15% of carbohydrates and from 35 to 50% of plant fibres.

The diet according to the present invention is characterized by totally or partially eliminating carbohydrates from overweight persons' diet. Said kind of diet is usually known in the art as ketogenic diet. The ketogenic diet satisfies always one's appetite and brings always to a loss in weight, but may also cause several side-effects such as, for instance, hyperuricemia, hyperglycemia, hypercholesterolemia, hypertriglyceridemia, hepatic-pancreatic alterations, mental disorders, etc.

The carbohydrate content of the food composition according to the present invention is preferably less than 10%, advantageously less than 5%.

The proteins are preferably selected from the group comprising gluten, soya proteins, milk proteins in particular from soya milk without lactose, animal proteins obtained from meat or dried or smoked fish, egg albumen and yolk, wheat proteins, wheat germ, rice germ, soya bean protein, pea protein.

The plant fibres are preferably selected from the group comprising cereal fibres, in particular wheat, maize and oats, wheat, maize and soya bean brands, vegetable fibres, in particular tomato, spinach, inulin, acacia and fruit fibres, in particular oranges and apples.

The food composition according to the present invention may additionally comprise vegetal fat, in particular from coconut and soya bean and animal lipids, in particular from yolk, cream and whole milk.
Said food composition may further comprise flours, in particular flours selected from the group consisting of tender wheat flour and drum wheat flour.

Both the proteins and the plant fibres are used in a finely divided form and mixed in suitable ratios to produce flours which can be used as substitutes for wheat flour in the preparation of foods such as pasta, bread, bread sticks, bakery products and pastries.

This invention also relates to a method for improving the appearance of a person by achieving a loss of weight which is beneficial from the aesthetic point of view, this method comprising the elimination from the said person's diet of all carbohydrate-based foods and their replacement with foods obtained using the flours described above.

Advantageously such a method provides for the initial use of flours having the lowest carbohydrate content possible, in any event a content of not more than 5% by weight.

A certain although minimum carbohydrate content is always present in the flours according to the invention because commercially available plant fibres always have a small residual glucide content. Once the desired aesthetic effects have been achieved, it is then possible, according to the method of the invention, to use flours with a higher carbohydrate content in a second stage. Preferably flours with a carbohydrate content up to 15% should be used.

In a third stage of the method, which can be described as a maintenance stage, the carbohydrate content of the flours can be further increased, without however exceeding the threshold of 20% by weight. This guarantees maintenance of the aesthetic effects obtained, while at the same time introducing greater variety into the diet.

In addition, the effect of the above diet can be improved by means of a combination with adjuvants for the treatment of obesity. Said adjuvants comprise at least one adjuvant selected from the group consisting of sedative-ansiolytic agents, anorectic agents and lipolytic agents.

The sedative-ansiolytic agent is preferably a benzodiazepine, most preferably 7-chloro-2,3-dihydro-2-oxo-5-phenyl-1H-1,4-benzodiazepine (dipotassium chlorazepate). A preferred dosage of the dipotassium chlorazepate is from 0,5 mg to 10 mg per day for an adult of a weight of about 70 kg.

The anorectic agent is preferably selected from the group consisting of 1-phenyl-2-diethylamine-1-proparone hydrochloride (diethylpropione chlorohydrate), fenfluramine chlorohydrate and D-fenfluramine chlorohydrate. A preferred dosage of the anorectic agent is from 2 mg to 35 mg per day for an adult of a weight of about 70 kg.

The lipolytic agent is preferably selected from the group consisting of analogues of tiroxine, most preferably said agent is 3,5,3'triiodiotiroacetic acid. A preferred dosage of the lipolytic agent is from 0,2 mg to 0,8 mg per day for an adult of a weight of about 70 kg.

The method according to the invention therefore provides for an individual who intends to lose weight to eliminate all carbohydrate-rich foods such as bread, pasta, sweets and bakery products in general from his diet and to replace them by "facsimile" foods obtained using the flours described above, using non-sugar sweeteners in the case of sweets.

The above-mentioned flours may be used in the same way as wheat flour. To prepare bread for example, such flours are made into a dough with water and yeast, and possibly salt, lard, olive oil or other optional ingredients, allowed to rise and then baked in an oven at the same temperature at which bread is baked.

Similarly, in order to prepare sweets such as tarts OR biscuits, the flours are made into a dough with butter or margarine or similar fats, eggs, yeast if desired and other optional ingredients, with the addition of non-glucide sweeteners (e.g. saccharine) and baked in an oven in the same way as traditional sweets.

Those who intend to retain their ideal weight by following the method according to the invention should also abstain from consuming fruits which are rich in sugars, sweetened drinks, etc.

The food composition and the method according to this invention will be further described with reference to the examples provided below merely by way of non-restrictive illustration in which the percentages stated are to be understood as percentages by weight of the total dry weight.

### EXAMPLE 1

| | |
|---|---|
| Wheat gluten | 55% |
| Wheat fibre | 40% |
| Vegetable fibres | 5% |

By wheat fibre is meant the fibre of the wheat stems (one example of a commercially available product is that sold by the name of VITACEL®).

Wheat gluten is a product marketed by various firms such as for example the company Rocchetta (Italy).

The vegetable fibres are for example fibres of tomato, spinach, onion, etc.; these fibres are marketed by various firms, including Newfood (Italia).

### EXAMPLE 2

| | |
|---|---|
| Wheat gluten | 35% |
| Soya proteins | 20% |
| Wheat fibre | 45% |

An example of a commercially available product comprising soya protein is soymin®.

### EXAMPLE 3

| | |
|---|---|
| Wheat gluten | 35% |
| Fruit fibres | 45% |
| Soya proteins | 20% |

The fruit fibres used in this composition were those marketed in Italy under the name VITACEL Arancia® and VITACEL Mela®.

### EXAMPLE 4

| | |
|---|---|
| Wheat gluten | 34% |
| Wheat germ | 33% |
| Wheat fibre | 17% |
| Vegetable fibres | 16% |

Wheat germ is marketed by various producers, such as for example the company Rizzolio (Italy).

### EXAMPLE 5

| | |
|---|---|
| Milk proteins | 10% |
| Wheat fibre | 15% |
| Wheat germ | 60% |
| Fruit fibres | 15% |

### EXAMPLE 6

The flour in Example 1 was used to prepare pasta. For this purpose the flour was mixed with a sufficient quantity of water to obtain a dough suitable for forming by extrusion (approximately 20 to 30% of water). A pasta in the form of spaghetti was obtained by extrusion through suitable dies and when subsequently cooked in boiling salted water yielded organoleptic properties entirely similar to those of a pasta made with high quality durum wheat semolina.

### EXAMPLE 7

The flour in Example 2 was used to prepare bread. For this purpose the flour was mixed with a sufficient quantity of water to obtain a mouldable dough, and to this a suitable quantity of bread yeast and salt were also added. The dough was then left to rise for approximately 2 hours, mixed again and finally left to stand for approximately 1 hour before being shaped and baked in an oven at approximately 220°C.

The bread obtained in this way had optimum organoleptic properties, crustiness and crumbliness.

The flours in the examples described above are virtually interchangeable and can be used in the preparation of pasta, egg pasta, bread, bread sticks, biscottes, sweet and savoury flans, biscuits, etc., following the addition of suitable additional ingredients.

The only differences which can be found between the various flours lie in their different carbohydrate contents, which for example are lowest in the flour according to Example 1 (definitely below 5%) and tend to be higher (although always below 15%) in the flours containing fruit or vegetable fibres, on account of the residual glucide content of those fibres.

As a consequence a person who wishes to lose weight by following the method according to this invention should initially use flours such as those in Examples 1 or 2, which guarantee an absolutely negligible glucide content.

Once the ideal weight has been gained the individual can alternate the use of the abovementioned flours with those of flours (such as those in Examples 3 and 4) which have a certain, though low, glucide content due to the fruit or vegetable fibre content.

Once the individual's weight and form have become stabilized, then flours containing other ingredients such as milk proteins, dried meat or fish animal proteins, powdered milk, powdered cream, whole eggs or dried albumin or dried egg yolk only can also be introduced into the diet.

In this latter stage the carbohydrate content should however always be maintained below 20% by weight.

It should be pointed out that the flours according to the invention constitute the basis of the food intake of an individual who intends to improve his appearance by eliminating excess kilos using the method according to this invention. Provided that all carbohydrate-based foods are replaced by similar foods prepared using the flours according to the invention the individual can nevertheless consume a variety of foods rich in proteins and/or lipids (meat, fish, delicatessen products, cheeses) without any limit on quantity and without in any way prejudicing the result achieved, or losing excessive weight.

This constitutes an enormous advantage in comparison with the low-calorie diet regimes proposed hitherto, which presuppose a considerable propensity to "sacrifice" on the part of individuals who subject themselves to them, and which for this reason have therefore as a result achieved very low compliance on the part of such individuals.

In addition to imposing no quantitative limits on food intake, the method according to the invention makes it possible to vary foods consumed with great freedom, which further increases the willingness of individuals to follow the proposed diet regime satisfactorily.

## Claims

1. A food composition in the form of a flour comprising, as percentages by weight of the total dry weight, at least 50% of protein, up to 20% of carbohydrates and from 30 to 50% of plant fibres.

2. A food composition according to claim 1, in which the carbohydrates content is up to 15%, preferably less than 5%.

3. A food composition according to claim 1 or 2, in which:
- the proteins are preferably selected from the group comprising gluten, soya proteins, milk proteins in particular from soya milk without lactose, animal proteins obtained from meat or dried or smoked fish, egg albumen and yolk, wheat proteins, wheat germ, rice germ, soya bean protein, pea protein; and
- the plant fibres are selected from the group comprising the fibres of cereals, in particular wheat, maize and oaths wheat, maize and soya bean brands, vegetable fibres, in particular tomato, spinach, inulin, acacia and fruit fibres, in particular oranges and apples, said food composition optionally comprising
- vegetal fat, in particular from coconut and soya bean and/or animal lipids, in particular from yolk, cream and whole milk, and/or
- flours, in particular flours selected from the group consisting of tender wheat flour and durum wheat flour.

4. A substitute food for pasta wherein the flour component is a flour according to any one of claims 1 to 3.

5. A substitute food for bread or similar bakery products wherein the flour component is a flour according to any one of claims 1 to

6. A method for improving the appearance of person by achieving a loss of weight which is beneficial from the aesthetic point of view, this method comprising the elimination from the said person's diet of all carbohydrate-based foods and their replacement with foods obtained using the food compositions according to any one of claims 1 to 3.

7. A method according to claim 6, **characterized in that** it comprises:
an initial stage in which the said carbohydrate-based foods are replaced with foods obtained using food compositions according to claim 2, in which the carbohydrate content is less than 5%;
an intermediate stage in which the said carbohydrate-based foods are replaced with foods obtained using food compositions according to claim 2 in which the carbohydrate content is up to 15%, and
a maintenance stage in which the said carbohydrate-based foods are replaced with foods obtained using food compositions according to claim 1.

8. Use of a food composition according to claims 1 to 3 in the preparation of a medicament for achieving the loss of weight.

9. Use according to claim 8 wherein said medicament comprises at least one adjuvant selected from the group consisting of a sedative-ansiolytic agent, an anorectic agent and a lipolytic agent.

10. Use according to claim 9, wherein said sedative-ansiolytic agent is preferably a benzodiazepine, most preferably dipotassium chlorazepate; said anorectic agent is preferably selected from the group consisting of diethylpropione chlorhydrate, fenfluramine chlorohydrate, D-fenfluramine chlorhydrate; said lipolytic agent is preferably selected from the group consisting of analogues of tiroxine, most preferably triiodiotiroacetic acid.

## Patentansprüche

1. Nahrungsmittelzusammensetzung in Form eines Mehles, umfassend, angegeben als Gewichtsprozentsätze, bezogen auf das Gesamttrockengewicht, mindestens 50 % Protein, bis zu 20 % Kohlenhydrate und 30 bis 50 % Pflanzenfasern.

2. Nahrungsmittelzusammensetzung nach Anspruch 1, in welcher der Kohlenhydratgehalt bis zu 15 %, vorzugsweise weniger als 5 % beträgt.

3. Nahrungsmittelzusammensetzung nach Anspruch 1 oder 2, in welcher:
die Proteine vorzugsweise ausgewählt sind aus der Gruppe umfassend Gluten, Sojaproteine, Milchproteine, insbesondere aus Sojamilch ohne Lactose, tierische Proteine, erhalten aus Fleisch oder aus getrocknetem oder geräuchertem Fisch, Eiweiß bzw. Eialbumin und Eidotter, Weizenproteine, Weizenkeim, Reiskeim, Sojabohnenprotein, Erbsenprotein; und
die Pflanzenfasem ausgewählt sind aus der Gruppe umfassend die Fasem von Getreide, insbesondere Weizen, Mais und Hafer, Weizen-, Mais- und Sojabohnensorten, pflanzliche Fasem, insbesondere Tomaten-, Spinat-, Inulin-, Akazien- und Fruchtfasem, insbesondere Orangen und Äpfel,
wobei die Nahrungsmittelzusammensetzung gegebenenfalls umfasst
pflanzliches Fett, insbesondere aus Kokosnuss und Sojabohnen und/oder tierische Lipide, insbesondere aus Eidotter, Sahne und Vollmilch und/oder
Mehle, insbesondere Mehle ausgewählt aus der Gruppe bestehend aus Weichweizenmehl und Hartweizenmehl.

4. Ersatznahrungsmittel für Teigwaren, worin die Mehlkomponente ein Mehl nach einem der Ansprüche 1 bis 3 ist.

5. Ersatznahrungsmittel für Brot oder ähnliche Backwaren, worin die Mehlkomponente ein Mehl nach einem der Ansprüche 1 bis 3 ist.

6. Verfahren zum Verbessern des Aussehens einer Person durch Erzielen einer Gewichtsabnahme, welche unter einem ästhetischen Gesichtspunkt günstig ist, wobei dieses Verfahren die Entfernung aller Nahrungsmittel auf Kohlenhydrat-Basis aus der Diät der Person und ihre Ersetzung durch Nahrungsmittel umfasst, die unter Verwendung der Nahrungsmittelzusammensetzungen nach einem der Ansprüche 1 bis 3 erhalten werden.

7. Verfahren nach Anspruch 6, **dadurch gekennzeichnet, dass** es umfasst:
ein Anfangsstadium, in welchem die Nahrungsmittel auf Kohlenhydrat-Basis durch Nahrungsmittel ersetzt werden, die unter Verwendung von Nahrungsmittelzusammensetzungen nach Anspruch 2 erhalten werden, in welchen der Kohlenhydratgehalt weniger als 5 % beträgt;
ein Zwischenstadium, in welchem die Nahrungsmittel auf Kohlenhydrat-Basis durch Nahrungsmittel ersetzt werden, die unter Verwendung von Nahrungsmittelzusammensetzungen nach Anspruch 2 erhalten werden, in welchen der Kohlenhydratgehalt bis zu 15 % beträgt, und
ein Aufrechterhaltungsstadium, in welchem die Nahrungsmittel auf Kohlenhydrat-Basis durch Nahrungsmittel ersetzt werden, die unter Verwendung von Nahrungsmittelzusammensetzungen nach Anspruch 1 erhalten werden.

8. Verwendung einer Nahrungsmittelzusammensetzung nach den Ansprüchen 1 bis 3 bei der Herstellung eines Medikaments zum Erzielen einer Gewichtsabnahme.

9. Verwendung nach Anspruch 8, wobei das Medikament mindestens ein Adjuvans, ausgewählt aus der Gruppe bestehend aus einem sedativen und angstlösenden Mittel, einem Appetitzügler und einem lipolytischen Mittel, umfasst.

10. Verwendung nach Anspruch 9, wobei das sedative und angstlösende Mittel vorzugsweise ein Benzodiazepin, am meisten bevorzugt Dikaliumchlorazepat ist; der Appetitzügler vorzugsweise ausgewählt ist aus der Gruppe bestehend aus Diethylpropion-hydrochlorid, Fenfluramin-hydrochlorid, D-Fenfluramin-hydrochlorid; das lipolytische Mittel vorzugsweise ausgewählt ist aus der Gruppe bestehend aus Analoga von Thyroxin, am meisten bevorzugt Triiodthyroessigsäure.

## Revendications

1. Composition alimentaire sous la forme d'une farine comprenant, en pourcentages du poids total à sec, au moins 50 % de protéines, jusqu'à 20 % de glucides et de 30 à 50 % de fibres végétales.

2. Composition alimentaire selon la revendication 1, dans laquelle la teneur en glucides atteint jusqu'à 15 %, de préférence moins de 5 %.

3. Composition alimentaire selon la revendication 1 ou 2 dans laquelle :
- les protéines sont choisies de préférence dans le groupe comprenant le gluten, les protéines de soja, les protéines de lait, en particulier de lait de soja sans lactose, les protéines animales obtenues à partir de viande ou de poisson séché ou fumé, l'albumine et le jaune d'oeuf, les protéines de blé, le germe de blé, le germe de riz, la protéine de graine de soja, la protéine de pois, et
- les fibres végétales sont choisies dans le groupe comprenant les fibres de céréales, en particulier de blé, de maïs et d'avoine, les sons de maïs et de graines de soja, les fibres de légumes, en particulier de tomate, d'épinard, d'inuline, d'acacia et les fibres de fruits, en particulier d'oranges et de pommes,
ladite composition alimentaire comprenant au choix
- de la graisse végétale, en particulier de noix de coco et de graines de soja, et/ou des lipides d'origine animale, en particulier de jaune d'oeuf, de crème et de lait entier et/ou
- des farines, en particulier des farines choisies dans le groupe constitué par la farine de blé tendre et la farine de blé dur.

4. Aliment de substitution pour pâtes alimentaires, dans lequel le composant farine est une farine selon l'une quelconque des revendications 1 à 3.

5. Aliment de substitution pour pain ou produits de boulangerie similaires, dans lequel le composant farine est une farine selon l'une quelconque des revendications 1 à 3.

6. Procédé pour améliorer l'aspect d'une personne en atteignant une perte de poids bénéfique du point de vue esthétique, ce procédé comprenant la suppression dans le régime alimentaire de ladite personne de tous les aliments à base de glucides et leur remplacement par des aliments obtenus en utilisant les compositions alimentaires selon l'une quelconque des revendications 1 à 3.

7. Procédé selon la revendication 6, **caractérisé en ce qu'**il comprend :
une étape initiale dans laquelle lesdits aliments à base de glucides sont remplacés par des aliments obtenus en utilisant des compositions alimentaires selon la revendication 2, dans lesquelles la teneur en glucides est inférieure à 5 % ;
une étape intermédiaire dans laquelle lesdits aliments à base de glucides sont remplacés par des aliments obtenus en utilisant des compositions alimentaires selon la revendication 2 dans lesquelles la teneur en glucides peut atteindre 15 %, et
une étape d'entretien dans laquelle les aliments à base de glucides sont remplacés par des aliments obtenus en utilisant des compositions alimentaires selon la revendication 1.

8. Utilisation d'une composition alimentaire selon les revendications 1 à 3 dans la préparation d'un médicament destiné à réaliser la perte de poids.

9. Utilisation selon la revendication 8, dans laquelle ledit médicament comprend au moins un adjuvant choisi dans le groupe constitué par un agent sédatif-anxiolytique, un agent anorexique et un agent lipolytique.

10. Utilisation selon la revendication 9, dans laquelle ledit agent sédatif-anxiolytique est de préférence une benzodiazépine, et de façon particulièrement préférée le chlorazépate de dipotassium ; ledit agent anorexique est de préférence choisi dans le groupe constitué par le chlorhydrate de diéthylpropione, le chlorhydrate de fenfluramine, le chlorhydrate de D-fenfluramine ; ledit agent lipolytique est choisi de préférence dans le groupe constitué par des analogues de la thyroxine, de façon particulièrement préférée l'acide tri-iodothyro-acétique.
